# EUROPÄISCHE PATENTANMELDUNG

(11) **EP 4 371 514 A1**
(43) Veröffentlichungstag der Anmeldung: **22.05.2024**
(21) Anmeldenummer: 22208602.7
(22) Anmeldetag: 21.11.2022
(51) Int. Cl.: A61B 18/14, A61B 18/00

(54) **MEDIZINISCHES THERMOFUSIONSINSTRUMENT**

(71) Anmelder: Erbe Elektromedizin GmbH, 72072 Tübingen (DE)
(72) Erfinder: FRANKE, Jennifer, 72108, Rottenburg (DE); KAUPP, Stefan, 72072, Tuebingen (DE); BOB, Felix, 72108, Rottenburg (DE)
(74) Vertreter: Rüger Abel Patentanwälte PartGmbB

(57) **Zusammenfassung**

Bei dem erfindungsgemäßen Thermofusionsinstrument wird eine Stromzuführungsanordnung (22) sowohl zur elektrischen Versorgung der beiden Elektroden (18, 19) wie auch zur mechanischen Kraftübertragung herangezogen. Durch die Ausbildung der beiden elektrischen Leiter (23, 28) als hochkant stehende Flachteile und die kräftemäßige Kopplung derselben in einer Verzahnungs (32) können die elektrischen Leiter (23, 28) als mechanische Aussteifungselemente genutzt werden, wobei ein filigraner, spaltfreier leicht und zuverlässig sterilisierbarer Aufbau erhalten wird. Die Stromversorgung des Instruments (10) erfolgt über lediglich ein einziges Kabel (20), das an nur eine der beiden Branchen (15, 16) vorgesehen ist.

## Beschreibung

Die Erfindung betrifft ein medizinisches Thermofusionsinstrument, wie es beispielsweise zum Versiegeln und gegebenenfalls auch Trennen von biologischem Gewebe, insbesondere Blutgefäßen und ähnlichen Hohlorganen, Anwendung finden kann.

Thermofusionsinstrumente werden bei operativen Eingriffen am Patienten eingesetzt und müssen danach entweder entsorgt oder auch gereinigt und sterilisiert werden. Letzteres stellt hohe Anforderungen an die mechanische, thermische und chemische Belastbarkeit des Instruments und an seinen möglichst spaltfreien Aufbau, um eine leichte und zuverlässige Reinigbarkeit zu ermöglichen. Trotz wiederholter Reinigungszyklen muss seine Funktion sichergestellt bleiben.

Thermofusionselemente sind grundsätzlich bekannt. Beispielsweise offenbart die EP 1 595 509 A2 ein solches zangenartiges Instrument, dessen beide Branchen bei einem Scharnier beweglich aneinander gelagert sind. Eine der Branchen dient der Stromzufuhr für beide bei der Thermofusion zusammenwirkenden Versiegelungselektroden. Während der elektrische Strom für eine der Elektroden in der betreffenden Branche direkt an die Elektrode weitergegeben werden kann, wird der Strom für die andere Elektrode über das Scharnier geleitet. Dazu ist im Scharnierbereich ein Isolierkörper mit einer Ringnut vorgesehen, in der ein Ringschleifkontakt für die andere Branche liegt.

Ein Thermofusionsinstrument mit Stromzufuhr durch eine der beteiligten Branchen ist ebenso aus der EP 3 744278 A1 bekannt. Die Branchen sind dort aus kunststoffummanteltem Metall hergestellt, sodass die beim Zusammenpressen von biologischem Gewebe nötigen mechanischen Kräfte von den Griffenden sicher auf das zangenartige Werkzeug übertragen werden können.

Eine weitere Variante solcher Instrumente ist aus der WO 2009/149799 A1 bekannt. Das Strom von einer Branche zu der anderen Branche übertragende Scharnier ist als Schalter ausgebildet, sodass die elektrisch leitende Verbindung erst beim Schließen des Instruments freigegeben wird.

Außerdem offenbart die DE 103 23 533 B4 ein Thermofusionsinstrument mit Stromzuführung zu beiden Elektroden über eine der beiden Branchen. Dabei wird der Metallkörper der Branche als Leiter für einen Pol und ein an der Branche vorgesehener Draht als zweiter Pol genutzt.

Schließlich ist aus der EP 1 153 578 B1 ein zangenartiges Thermofusionsinstrument bekannt, bei dem ein Scharnierbolzen vorgesehen ist, um Strom von einem aus Blech ausgebildeten Leiter durch das Scharnier hindurch zu der anderen Branche zu übertragen.

Bei der Gestaltung eines medizinischen Thermofusionsinstruments kommt es neben der Sterilisierbarkeit und Wiedersterilisierbarkeit auch auf eine hohe Präzision und hohe auch mechanische Belastbarkeit an. Insbesondere sollen solche Instrumente auch möglichst filigran gestaltet werden, ohne Abstriche an der Stabilität sowie der Dichtigkeit und dem Schutz etwaiger elektronischer Komponenten zu machen, die in dem Instrument angeordnet sein können.

Deswegen ist es Aufgabe der Erfindung, ein Konzept für ein medizinisches Thermofusionsinstrument anzugeben, das dank seiner Grundstruktur und seines praktischen Aufbaus verlässlich sterilisierbar und dabei filigran und bequem in der Handhabung ist.

Diese Aufgabe wird mit den Thermofusionsinstrument nach Anspruch 1 gelöst:

Das erfindungsgemäße Thermofusionsinstrument weist zwei Branchen auf, die an einem Scharnier schwenkbar aneinander gelagert sind. Lediglich eine der beiden Branchen ist mit einem Kabel versehen und weist eine Stromzuführungsanordnung auf, über die die beiden Elektroden mit Strom und Spannung versorgbar sind. Zu der Stromzuführungsanordnung gehören zwei Leiter, die durch flache, bandförmige Elemente gebildet sind. Die Elemente können ganz oder teilweise als Blechelemente oder Feingusselemenete ausgebildet sein. Dabei ist eines der Elemente eben und das andere der Elemente zwar ebenfalls weitgehend eben, jedoch an einer Stelle gekröpft ausgebildet. Dadurch können die beiden durch die flachen Elemente gebildeten Leiter in einem ersten Abschnitt in der Nähe des Scharniers in einer gemeinsamen Ebene liegend angeordnet sein, wobei sie bei einer mit festem, elektrisch isolierendem Material gefüllten Fuge aneinander grenzen. In einem anderen, sich von den Scharnier weiter fort zu einem Griffende der Branche erstreckenden zweiten Abschnitt können die beiden Leiter mit ihren Flachseiten aufeinander zuweisend im Abstand nebeneinander angeordnet sein. Sie können dabei in der Branche vorzugsweise hochkant, d.h. innerhalb von Ebenen angeordnet sein, auf denen die Scharnierachse senkrecht steht. Dies ermöglicht die Nutzung der beiden flachen Elemente nicht nur zur Stromübertragung, sondern auch zur Erzeugung der gewünschten Biegesteifigkeit der betreffenden Branche, die ansonsten aus Kunststoff besteht.

Die Fuge, an der die beiden Abschnitte der Elemente aneinander grenzend angerordnet sind, dient dabei der Kraftübertragung zwischen den Elementen innerhalb der Branche. Diese wird dadurch ausgesteift und erlangt eine große Biegebelastbarkeit. Das biologische Gewebe kann dadurch fest gegriffen und Hohlorgane können sicher zusammengedrückt werden können, ohne das Thermofusionsinstrument auch bei wiederholten Gebrauch und nach mehrmaliger Sterilisierung zu überlasten. So sind stets gute Fusionsergebnisse erreichbar.

Der erste Leiter und die mit ihm elektrisch verbundene erste Elektrode können nahtlos einstückig ausgebildet sein. Durch das Fehlen von Kontaktstellen ist inhärent Zuverlässigkeit gegeben.

Das Scharnier ist vorzugsweise elektrisch leitfähig ausgebildet, um den zweiten Leiter elektrisch mit der zweiten Elektrode zu verbinden. Dazu weist das Scharnier vorzugsweise zwei Scharnierhälften auf, die an einander zugeordneten Kontaktflächen in elektrischer und reibschlüssiger Berührung stehen. Es ist möglich, den Kontaktflächen ein oder mehreren Federelemente zuzuordnen, um einen ständigen elektrischen Kontakt zwischen ihnen sicherzustellen. Vorzugsweise ist zumindest eine der Kontaktflächen der beiden Scharnierhälften kegelstumpfförmig ausgebildet, was eine gute Kontaktgabe ermöglicht. Vorzugsweise sind die Kontaktflächen der beiden Scharnierhälften zueinander passend kegelstumpfförmig ausgebildet.

Bei einer bevorzugten Ausführungsform ist die Fuge zwischen den beiden Elementen auf spezielle, insbesondere hinsichtlich der Kraftübertragung vorteilhafte Weise ausgebildet. Dazu kann die Fuge einen Verzahnungsabschnitt aufweisen, bei dem eines der Elemente eine Ausnehmung und das andere der beiden Elemente einen in die Ausnehmung passenden Vorsprung aufweist. Die Ausnehmung und der Vorsprung liegen jedoch nicht aneinander an, sondern definieren eine entsprechende Fuge. Die Fuge ist vorzugsweise Ω-förmig ausgebildet, was bei einerseits guter elektrischer Isolation andererseits eine gute Kraftübertragung zwischen den Elementen möglich macht.

Es ist vorteilhaft, wenn der Vorsprung scheibenförmig ausgebildet ist und einen Halsabschnitt aufweist, über den er mit dem übrigen Element nahtlos einstückig verbunden ist. Der Halsabschnitt ist dabei vorzugsweise schmaler als der scheibenförmige (Kopf-)Abschnitt, wodurch eine formschlüssige Verzahnung zwischen den beiden flachen Elementen erreicht werden kann. Der scheibenförmige Kopf kann einen kreisbogenförmigen oder auch anderweitig geformten Rand aufweisen.

In dem zweiten sich von dem Scharnier zu dem Handgriff erstreckenden Abschnitt der Branche sind die beiden Elemente vorzugsweise in einen Isolierkörper eingebracht. Dieser Isolierkörper sichert die Position der Elemente zueinander vor dem Umspritzen mit weiterem Kunststoff, der letztendlich die Fuge in dem Verzahnungsabschnnitt füllt und die Elemente zueinander lagerichtig fixiert. Die exakte Positionierung der Elemente zueinander kann insbesondere auch von den Scharnierhälften sichergestellt werden, die als entsprechende Metallelemente mit den beteiligten Elementen vor dem Umspritzen mit Kunststoff verschweißt sind und die in einer Spritzgussform exakt positionierbar sind.

Ausführungsformen der Erfindung ergeben sich aus der Zeichnung sowie der zugehörigen Beschreibung. Es zeigt:
Figur 1 eine Ausführungsform des Thermofusionsinstruments, in perspektiver Übersichtsdarstellung,
Figur 2 Elektroden und die zugehörige Stromzuführungsanordnung insbesondere im Scharnierbereich, in perspektivischer Explosionsdarstellung,
Figur 3 einen Fugenabschnitt zwischen Leiterelementen der Stromzuführungsanordnung nach Figur 2, in ausschnittsweiser Darstellung,
Figur 4 Scharnierhälften des Scharniers des Thermofusionsinstruments, in Seitenansicht,
Figur 5 die Stromzuführungsanordnung des erfindungsgemäßen Thermofusionsinstruments in Draufsicht,
Figur 6 Leiterelemente der Stromzuführungsanordnung, in Draufsicht und Explosionsdarstellung,
Figur 7 eine Querschnittsdarstellung des Griffbereichs der Branche mit Stromzuführungsanordnung.

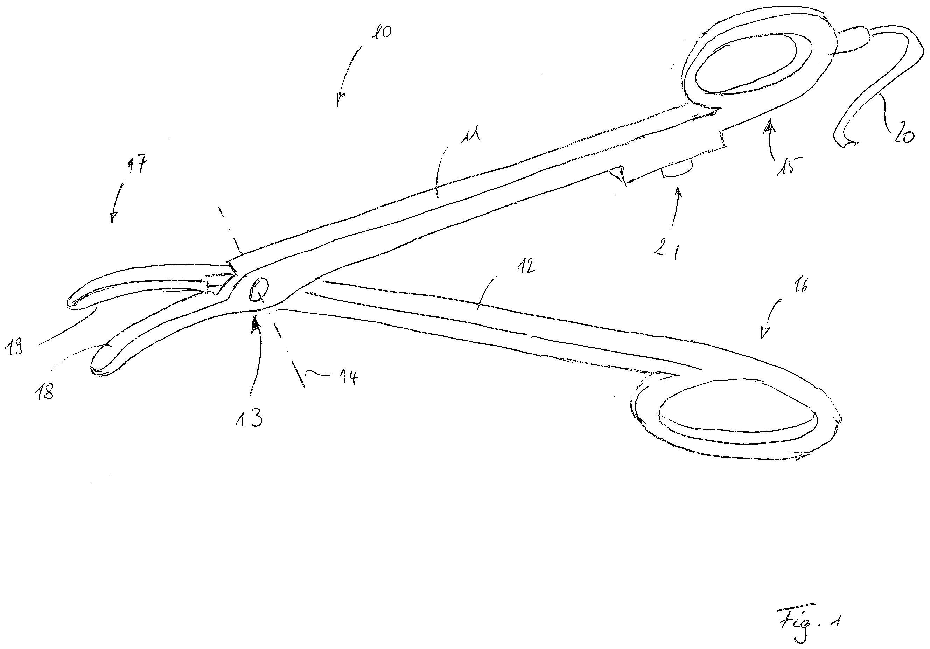

Figur 1 veranschaulicht ein wiederholt reinigbares und wiedersterilisierbares Thermofusionsinstrument 10, das als Gewebezange zum Fusionieren von biologischem Gewebe ausgebildet ist. Es weist eine erste Branche 11 und eine zweite Branche 12 auf, die bei einem Scharnier 13 um eine Scharnierachse 14 schwenkbar aneinander gelagert sind. Die beiden Branchen 11, 12 weisen bezüglich des Scharniers 13 proximal Griffenden 15, 16 und bezüglich des Scharniers 13 distal ein zangenartiges Werkzeug 17 auf. Dieses weist an aufeinander zuweisenden Flächen eine zu der ersten Branche 11 gehörige erste Elektrode 18 und eine zu der zweiten Branche 12 gehörige zweite Elektrode 19 auf. Die Elektroden 18, 19 dienen dazu, in dem Werkzeug 17 gefasstes und komprimiertes Gewebe zu bestromen, um es dadurch zu erwärmen und dabei eine Gewebefusion herbeizuführen.

Zur Stromversorgung der beiden Elektroden 18, 19 gehört ein mehradriges Stromzuführungskabel 20, das mit lediglich der ersten Branche 11 verbunden ist. Die zweite Branche 12 weist hingegen keinen Kabelanschluss auf.

An der ersten Branche 11 können Schaltmittel angeordnet sein, wie beispielsweise ein Schalter 21, der beim Schließen der Branchen 11, 12 einen Stromfluss zu den Elektroden 18, 19 freigibt, d.h. diese mit einem Generator verbindet, an den das Thermofusionsinstrument 10 über das Stromzuführungskabel 20 angeschlossen ist. In oder bei dem Schalter 21 kann eine elektronische Schaltung vorgesehen sein, um den Betrieb des Thermofusionsinstruments 10 auf geeignete Weise zu steuern oder zu beeinflussen. Beispielsweise kann die elektronische Schaltung die Bestromung der Elektroden 18, 19 in bestimmten Situationen abzuschwächen oder unterbrechen, z.B., wenn ein ausreichendes Fusionsergebnis erzielt ist.

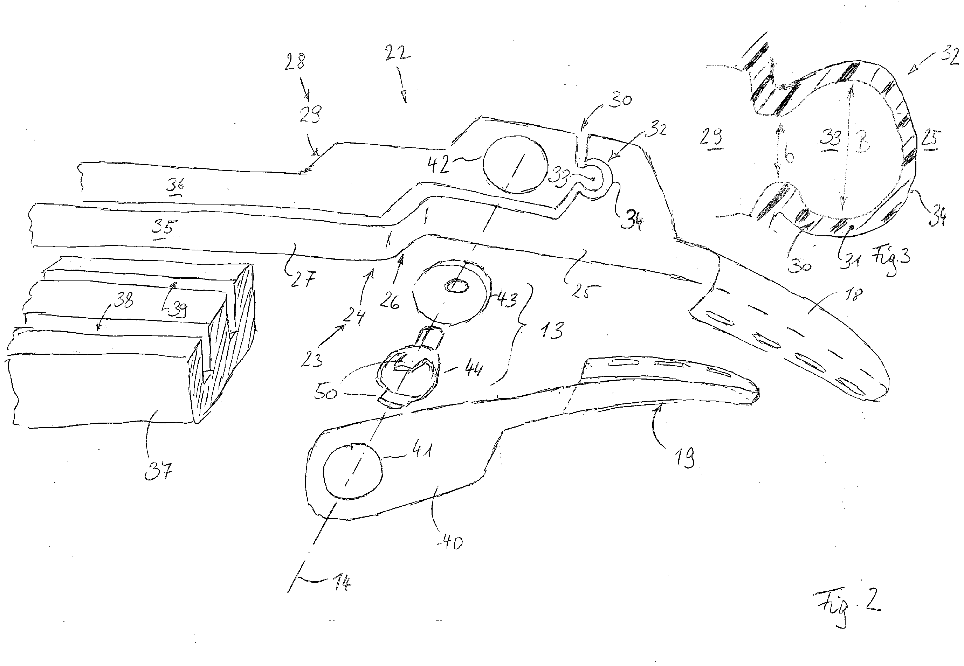

Die Branchen 11, 12 des Thermofusionsinstruments 10 bestehen vorwiegend aus Kunststoff, wobei in der ersten Branche 11 eine Stromzuführungsanordnung 22 angeordnet ist, die zumindest ausschnittsweise gesondert aus Figur 2 hervorgeht. Die Branche 12 kann ein Aussteifungselement aus Metall enthalten. In der Branche 11 dient hingegen die Stromzuführungsanordnung 22 als Aussteifungselement.

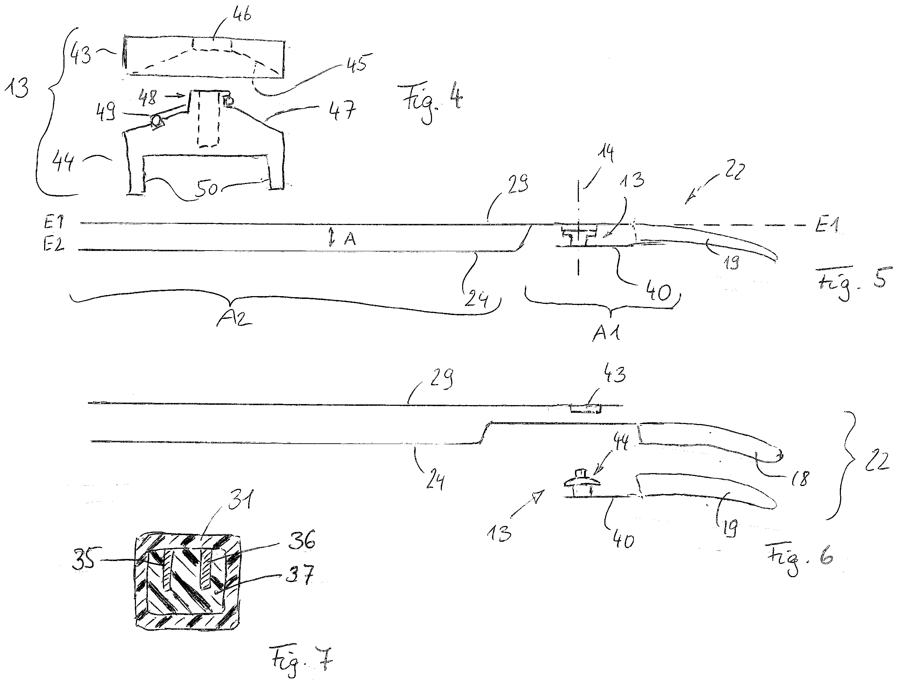

Zu der Stromzuführungsanordnung 22 gehört ein erster Leiter 23, der vorzugsweise durch ein streifenförmiges flaches Element 24, z.B. ein Blechelement gebildet ist. Das Element 24 kann aus Blech, alternativ aber auch ganz oder teilweise aus Guss bestehen. Dieses Element 24 umfasst die erste Elektrode 18, an die sich proximal ein ebener Abschnitt 25 anschließt. Dieser Abschnitt 25 liegt in einer Ebene E1 (siehe Figur 5), auf der Scharnierachse 14 senkrecht steht. Dieser Abschnitt 25 erstreckt sich um das Scharnier 13 herum und in proximaler Richtung etwas über die Scharnierachse 14 hinaus. Er geht dann an einer Kröpfung 26 in einen zweiten Abschnitt 27 des Leiters 23 über, der wiederum in einer zu der vorgenannten Ebene E1 parallelen zweiten Ebene E2 liegt, auf der die Scharnierachse 14 ebenfalls im Wesentlichen senkrecht steht.

Zu der Stromzuführungsanordnung 22 gehört außerdem ein zweiter Leiter 28, der als flaches ebenes Element 29 ausgebildet ist. Das Element 29 kann aus Blech, alternativ aber auch ganz oder teilweise aus Guss bestehen. Dieses Element 29 liegt zumindest im Bereich des Scharniers 13 in einer gemeinsamen Ebene mit dem Abschnitt 25 des Leiters 23, d.h. in der Ebene E1. Das Element 29 definiert hier mit dem Abschnitt 25 des Leiters 23 eine Fuge 30, die vergrößert gesondert in Figur 3 veranschaulicht ist. Die Fuge 30 ist mit Kunststoff 31 gefüllt, der bei der Herstellung der ersten Branche 11 um die Stromzuführungsanordnung 22 gespritzt wird und dabei in die Fuge 30 eindringt und diese füllt.

Die Fuge 30 hat zumindest eine solche Weite, dass die elektrische Durchschlagfestigkeit des Kunststoffs 31 in der Fuge 30 (und auch an anderer Stelle) bei den verwendeten Koagulationsspannungen nicht überschritten wird. Die Spannungsfestigkeit liegt vorzugsweise im Bereich von mehreren hundert Volt und überschreitet beispielsweise 500 V, um die nötige Betriebssicherheit sicherzustellen. Vorzugsweise ist die Weite der Fuge, d.h. der Abstand zwischen den Ementen 24, 29 in der Fuge 30 (und auch an anderer Stelle), zumindest so groß, wie die senkrecht zu ihren Flachseiten zu messende Dicke der Elemente 24, 29, d.h. z.B. die Dicke des verwendeten Blechs.

Vorzugsweise weist die Fuge 30 eine oder mehrere Verzahnungen 32 auf, wie sie aus Figur 2 hervorgeht und in Figur 3 gesondert dargestellt ist. Die Verzahnung 32 wird durch einen Vorsprung 33 des einen Elements, beispielsweise des Elements 29 gebildet, der in eine Ausnehmung 34 des anderen Elements, beispielsweise des Elements 24, greift. Die Fuge 30 kann in diesem Bereich zum Beispiel Ω-förmig, schwalbenschwanzförmig, schwalbenschwanzförmig gerundet oder ähnlich ausgebildet sein. Vorzugsweise weist der Vorsprung 33 eine maximale Breite B (Figur 3) auf, die größer ist als die Breite b dieses Vorsprungs in einem Steg oder Anschlussbereich, bei dem der vorzugsweise scheibenförmige Vorsprung 33 in das übrige Element 29 übergeht. Vorzugsweise ist der Vorsprung 33 einstückiger Bestandteil des Elements 29.

Während in Figur 2 und 3 der Vorsprung 33 dem Element 29 und die Ausnehmung 34 dem Element 24 zugeordnet ist, kann die Zuordnung auch umgekehrt getroffen sein. Es ist darüber hinaus möglich, mehrere solcher Vorsprung/Ausnehmung-Paarungen mit gleicher oder wechselseitige Orientierung vorzusehen.

Während die betreffenden Abschnitte der Elemente 24, 29 im Bereich des Scharniers 13 in der gemeinsamen Ebene E1 liegend angeordnet sind, sind sie in einem zweiten Abschnitt proximal bezüglich der Scharnierachse 14 parallel zueinander im Abstand A (siehe Figur 5) angeordnet, sodass sie mit zwei ihrer Flachseiten aufeinander zu und mit den jeweiligen anderen Flachseiten voneinander weg weisen. Die betreffenden Abschnitte 35, 36 der beiden Leiter 23, 28 können zur Isolierung und Distanzierung in einen entsprechenden Isolierkörper 37 beispielsweise aus Kunststoff eingelegt sein. Dieser kann dazu entsprechende Aufnahmeschlitze 38, 39 aufweisen, die sich längs durch den Isolierkörper 37 erstrecken.

Der zweite Leiter 28 ist über das Scharnier 13 mit der zweiten Elektrode 19 verbunden, die als Blechbiegeteil, als Gussteil (z.B. Feingussteil oder dergleichen) ausgebildet sein kann und einen flachen, sich zu dem Scharnier 13 erstreckenden Abschnitt 40 aufweist. Der Abschnitt 40 weist eine Öffnung 41 auf, die konzentrisch zu der Scharnierachse 14 ausgerichtet ist. Ebenso weist das Element 29 eine zu der Scharnierachse 14 konzentrische Öffnung 42 auf.

Zu dem Scharnier 13 gehören eine erste Scharnierhälfte 43 und eine zweite Scharnierhälfte 44, die vorzugsweise aus elektrisch leitfähigem Material, insbesondere Metall ausgebildet sein können, um eine elektrische Verbindung zwischen dem zweiten Leiter 28 und dem Element 40 zu schaffen. Die Scharnierhälften 43, 44 bilden ein elektrisch leitfähiges Schwenklager, das die Scharnierachse 14 festlegt.

Der Aufbau des Scharniers 13 geht aus Figur 2 und genauer aus Figur 4 hervor:
Die Scharnierhälfte 43 wird durch einen flachen zylindrischen Körper gebildet, in dem eine Sitzöffnung 45 ausgebildet sein kann. Die Sitzöffnung weist vorzugsweise eine Kegelstumpfform auf, die in einen Zylinderabschnitt 46 übergeht. Ist das Element 29 ein Blechteil, ist die erste Scharnierhälfte 43 ist mit einem der Elemente 29 oder 40, hier im Beispiel mit dem Rand der Öffnung 42 des Blechteils verschweißt oder anderweitig elektrisch leitend mechanisch fest verbunden. Ist das Element 29 wenigstens im Bereich des Scharbniers als Gussteil ausgebildet, kann die Scharnierhälfte 43 einstückig mit diesem als Bestandteil des Gussteils ausgebildet sein.

Die zweite Scharnierhälfte 44 weist eine zu der Kontaktfläche 45 komplementäre Kontaktfläche 47 auf, die in einen zylindrischen Fortsatz 48 übergehen kann. Dieser kann mit einer Sicherungsbohrung versehen sein, die der Aufnahme eines nicht weiter veranschaulichten Sicherungselements dient, um das Scharnier 13 in montiertem Zustand zu sichern.

Optional können in oder an der Kontaktfläche 45 und/oder der Kontaktfläche 47 ein oder mehrere Federmittel 49 angeordnet sein, beispielsweise ein Federring, der in einer entsprechend geformten Nut, hier beispielsweise einer Nut in der Kontaktfläche 47 und/oder dem Fortsatz 48 angeordnet ist.

Figur 5 veranschaulicht das erste Element 24, das zweite Element 29, das Scharnier 13 und das Element 40 in Draufsicht. Ersichtlich liegen die Elemente 24, 29 im Bereich des Scharniers 13 in der gemeinsamen Ebene E1, während sie in einem zweiten, proximal von dem Scharnier 13 beabstandeten Abschnitt in zueinander parallelen Ebenen E1, E2 angeordnet sind.

Zur besseren Verdeutlichung des Aufbaus des Scharniers 13 und der Elemente 29, 24, 40 wird ergänzend auf Figur 6 verwiesen. Wie ersichtlich ist die erste Scharnierhälfte 43 mit dem ersten Element 29 verbunden, während die zweite Scharnierhälfte 44, beispielsweise über zwei Fortsätze 50, mit dem Element 40 verbunden ist. Zwischen den beiden Fortsätzen 50 besteht ein Abstand, der durch weitere Elemente genutzt werden kann. Beispielsweise kann in dem Thermofusionsinstrument 10 ein Messerkanal vorgesehen sein, der sich durch das Scharnier 13 erstreckt. Beispielsweise kann ein solches Messer in Distalrichtung vorschiebbar, d.h. in dem Werkzeug 17 translatorisch beweglich angeordnet sein, um fusioniertes Gewebe in dem Werkzeug 17 zu durchtrennen.

Ist das Element 40 ein Blechteil, kann die Scharnirhälfte 44 mit dem Element 40 verschweißt werden, z.B. indem die Fortsätze 50 mit dm Rand der Öffnung 41 verschweißt werden. Alternativ können das Element 40 und die Scharnierhälfte 44 als ein Gussteil, z.B. Feingussteil bereitgestellt werden. Sie können durch ein einziges Gussteil oder durch miteinander nachträglich verbundene Gussteile hergestellt werden.

Zur Fertigung des Fusionsinstruments 19 können zunächst die Elemente 24, 29, 40 bereitgestellt werden. Sofern nicht schon vorhanden, werden in einem zweiten Schritt die Scharnierhälften 43, 44 an den Elementen 29, 40 erzeugt oder mit diesen verbunden. Die Elemente 24, 29 werden dann in den Isolierkörper 37 eingelegt. Alternativ wird der Isolierkörper an den Elementen 24, 29 oder um diese herum z.B. durch Urformen erzeugt. Die insoweit aus dem Scharnier 13, der Stromzuführungsanordnung 22 und dem Isolierkörper 37 gebildete Einheit wird dann in eine Spritzgussform eingelegt und im Ganzen mit dem Kunststoff 31 ummantelt, der dann auch, wie oben erwähnt, die Fuge 30 füllt. Es ergibt sich der Querschnitt durch die Branche 11, wie in Figur 7 dargestellt.

Die resultierende Branche 11 ist spaltfrei ausgebildet und kann trotz Verwendung von Kunststoff als Grundmaterial filigran und dabei mechanisch stabil ausgebildet werden. Die Stromzuführungsanordnung 22 übernimmt eine Doppelfunktion, nämlich die Stromzuleitung zu den Elektroden 18, 19 und die mechanische Aussteifung der Branche 11. Die von den Griffenden 15, 16 ausgehende Betätigungskraft wird von den beiden Elementen 24, 29 in Richtung des Werkzeugs 17 übertragen. Der über das Element 29 geleitete Kraftanteil wird über den Verzahnungsabschnitt 32 von dem Element 29 auf den Abschnitt 25 des Elements 24 und somit letztendlich auf die Elektrode 18 übertragen.

Bei dem erfindungsgemäßen Thermofusionsinstrument wird eine Stromzuführungsanordnung 22 sowohl zur elektrischen Versorgung der beiden Elektroden 18, 19 wie auch zur mechanischen Kraftübertragung herangezogen. Durch die Ausbildung der beiden elektrischen Leiter 23, 28 als hochkant stehende Flachteile und die kräftemäßige Kopplung derselben in einer Verzahnung 32 können die elektrischen Leiter 23, 28 als mechanische Aussteifungselemente genutzt werden, wobei ein filigraner, spaltfreier leicht und zuverlässig sterilisierbarer Aufbau erhalten wird. Die Stromversorgung des Instruments 10 erfolgt über lediglich ein einziges Kabel 20, das an nur eine der beiden Branchen 15, 16 vorgesehen ist.

### Bezugszeichen:

- 10: Thermofusionsinstrument
- 11: erste Branche
- 12: zweite Branche
- 13: Scharnier
- 14: Scharnierachse
- 15, 16: Griffenden
- 17: Werkzeug
- 18: erste Elektrode
- 19: zweite Elektrode
- 20: Stromzuführungskabel
- 21: Schalter
- 22: Stromzuführungsanordnung
- 23: erster Leiter
- 24: erstes Eelement
- 25: Abschnitt des Leiters 23 und Elements 24
- 26: Kröpfung des Elements 24
- 27: zweiter Abschnitt des Leiters 23 und Elements 24
- 28: zweiter Leiter
- 29: zweites Element
- 30: Fuge
- 31: Kunststoff
- 32: Verzahnungsabschnitt
- 33: Vorsprung
- 34: Ausnehmung
- 35: zweiter Abschnitt des ersten Leiters 23 / Elements 24
- 36: zweiter Abschnitt des zweiten Leiters 28 / Elements 29
- 37: Isolierkörper
- 38: Aufnahmeschlitz für den Abschnitt 35 des ersten Leiters 23
- 39: Aufnahmeschlitz für den Abschnitt 36 des zweiten Leiters 28
- 40: Element mit Elektrode 19
- 41: Öffnung in dem Abschnitt 40
- 42: Öffnung in dem Element 29
- 43: erste Scharnierhälfte
- 44: zweite Scharnierhälfte
- 45: Sitzöffnung und Kontaktfläche
- 46: Zylinderabschnitt
- 47: Kontaktfläche
- 48: Fortsatz
- 49: Federmittel
- 50: Fortsätze der zweiten Scharnierhälfte 44

## Patentansprüche

1. Medizinisches Thermofusionsinstrument (10):
mit einer ersten Branche (15) und einer zweiten Branche (16), die an einem Scharnier (13) schwenkbar aneinander gelagert sind, das eine Scharnierachse (14) festlegend ausgebildet ist,
mit einer in der ersten Branche (15) angeordneten Stromzuführungsanordnung (22), die einen ersten Leiter (23) und einen zweiten Leiter (28) umfasst, wobei der erste Leiter (23) mit einer an der ersten Branche (15) vorgesehenen ersten Elektrode (18) und der zweite Leiter (28) mit einer an der zweiten Branche (16) vorgesehenen zweiten Elektrode (19) elektrisch verbunden ist,
**dadurch gekennzeichnet,**
**dass** die beiden Leiter (23) durch bandförmige Elemente (24, 29) gebildet sind und
**dass** die Stromzuführungsanordnung (22) einen ersten Bereich (A1) aufweist, in dem Abschnitte (25) der beiden Leiter (23, 28) in einer gemeinsamen Ebene (E1) angeordnet sind, innerhalb derer sie bei einer mit festem, elektrisch isolierendem Material (31) gefüllten Fuge (30) aneinander grenzend angeordnet sind.

2. Thermofusionsinstrument nach Anspruch 1, **dadurch gekennzeichnet, dass** die Stromzuführungsanordnung (22) einen zweiten Abschnitt (A2) aufweist, in dem die beiden Leiter (23, 29) mit ihren Flachseiten im Abstand (A) nebeneinander angeordnet sind.

3. Thermofusionsinstrument nach Anspruch 2, **dadurch gekennzeichnet, dass** jeder Leiter (23, 29) in dem zweiten Abschnitt (A2) jeweils in einer Ebene (E1, E2) liegend angeordnet ist, zu denen die Scharnierachse (14) senkrecht orientiert ist.

4. Thermofusionsinstrument nach einem der vorstehenden Ansprüche, **dadurch gekennzeichnet, dass** der erste Leiter (23) und und die mit ihm elektrisch verbundene erste Elektrode (18) nahtlos einstückig ausgebildet sind.

5. Thermofusionsinstrument nach einem der vorstehenden Ansprüche, **dadurch gekennzeichnet, dass** das Scharnier (13) elektrisch leitfähig ausgebildet ist und dass der zweite Leiter (28) mit dem Scharnier (13) elektrisch verbunden ist.

6. Thermofusionsinstrument nach einem der vorstehenden Ansprüche, **dadurch gekennzeichnet, dass** die zweite Elektrode (19) mit dem Scharnier (13) elektrisch verbunden ist.

7. Thermofusionsinstrument nach einem der vorstehenden Ansprüche, **dadurch gekennzeichnet, dass** das Scharnier (13) zwei Scharnierhälften (43, 44) aufweist, die an einander zugeordneten Kontaktflächen (45, 47) in elektrischer und reibschlüssiger Berührung stehen.

8. Thermofusionsinstrument nach Anspruch 7, **dadurch gekennzeichnet, dass** eine der Kontaktflächen (45, 47) kegelstumpfförmig konkav ausgebildet ist.

9. Thermofusionsinstrument nach Anspruch 7 oder 8,
**dadurch gekennzeichnet, dass** eine der Kontaktflächen (45, 47) kegelstumpfförmig konvex ausgebildet ist.

10. Thermofusionsinstrument nach einem der Ansprüche 7 bis 9, **dadurch gekennzeichnet, dass** zwischen den Kontaktflächen (45, 47) ein Federelement (49) angeordnet ist.

11. Thermofusionsinstrument nach einem der vorstehenden Ansprüche, **dadurch gekennzeichnet, dass** die Fuge (30) eine Verzahnungsabschnitt (32) aufweist, bei dem eines der Elemente (24, 29) eine Ausnehmung (34) und das andere der beiden an die Fuge (30) grenzenden Elemente (24, 29) einen in die Ausnehmung (34) passenden Vorsprung (33) aufweist.

12. Thermofusionsinstrument nach Anspruch 11, **dadurch gekennzeichnet, dass** der Vorsprung (33) scheibenförmig ausgebildet ist und einen Halsabschnitt aufweist, über den er mit dem übrigen Element (29) nahtlos einstückig verbunden ist.

13. Thermofusionsinstrument nach Anspruch 12, **dadurch gekennzeichnet, dass** der Halsabschnitt eine Breite (b) aufweist, die geringer ist als eine in gleicher Richtung gemessene maximale Breite (B) des Vorsprungs (33) .

14. Thermofusionsinstrument nach einem der vorstehenden Ansprüche 2 bis 13, **dadurch gekennzeichnet, dass** die beiden Elemente (24, 29) in dem zweiten Abschnitt (A2) in einen Isolierkörper (37) eingelegt sind.

15. Thermofusionsinstrument nach einem der vorstehenden Ansprüche, **dadurch gekennzeichnet, dass** die Eelemente (24, 29) von einer Kunststoffmasse (31) umspritzt sind.
